# EUROPEAN PATENT SPECIFICATION

(11) **EP 1 545 569 B1**
(45) Date of publication and mention of the grant of the patent: **14.12.2016**
(21) Application number: 03748100.9
(22) Date of filing: 26.09.2003
(51) Int. Cl.: A61K 36/00

(54) **COMPOSITION COMPRISING PANAX GINSENG AND PAULLINIA CUPANA EXTRACTS**
ZUSAMMENSETZUNG, DIE PANAX GINSENG UND PAULLINIA CUPANA EXTRAKTE ENTHALTEN
PRODEDE DESTINE A AMELIORER UN APPRENTISSAGE COGNITIF

(30) Priority: 28.09.2002 DE 20215011 U; 12.03.2003 EP 03005568
(43) Date of publication of application: 29.06.2005
(73) Proprietor: Pharmaton S.A., 6934 Bioggio (CH)
(72) Inventor: GIANESELLO, Valter, CH-6945 ORIGLIO (CH); SOLDATI, Fabio, CH-6942 SAVOSA (CH); VIGNUTELLI, Alberto, CH-6900 LUGANO (CH); PETERS, Markus, 96740 Kailua Kona, HI (US)
(74) Representative: Simon, Elke Anna Maria
(86) International application number: PCT/EP2003/010749
(87) International publication number: WO 2004/028550

(56) References cited:
- EP-A1- 1 179 300
- WO-A-01/05253
- DE-A- 10 140 320
- DE-A- 10 140 320
- FR-A- 2 710 267
- FR-A- 2 710 267
- FR-A- 2 712 191
- FR-A- 2 712 191
- GB-A- 2 285 578
- US-A1- 2002 136 782
- PETKOV V D ET AL: "MEMORY EFFECTS OF STANDARDIZED EXTRACTS OF PANAX GINSENG (G115), GINKGO BILOBA (GK501) AND THEIR COMBINATION GINCOSAN (PHL-00701)" PLANTA MEDICA, THIEME, STUTTGART, DE, vol. 59, no. 2, 1993, pages 106-114, XP001029819 ISSN: 0032-0943
- BELCHEVA S ET AL: "GINKGO BILOBA L. AND PANAX GINSENG C.A. MEY. AS MEMORY-ENHANCING AND ANTI-ANXIETY DRUGS" PROCEEDINGS OF THE BIENNIAL CONFERENCE OF THE CANADIAN SOCIETY FOR COMPUTATIONAL STUDIES OF INTELLIGENCE, XX, XX, vol. 47, no. 2, 1994, pages 121-124, XP009011374
- ESPINOLA E.B. ET AL: "Pharmacological activity of Guarana ( Paullinia cupana Mart.) in laboratory animals." JOURNAL OF ETHNOPHARMACOLOGY, 55/3 (223-229)., 1977, XP001159800
- PETKOV V D ET AL: "MEMORY EFFECTS OF STANDARDIZED EXTRACTS OF PANAX GINSENG (G115), GINKGO BILOBA (GK501) AND THEIR COMBINATION GINCOSAN (PHL-00701)" PLANTA MEDICA, THIEME, STUTTGART, DE, vol. 59, no. 2, 1993, pages 106-114, XP001029819 ISSN: 0032-0943
- BELCHEVA S ET AL: "GINKGO BILOBA L. AND PANAX GINSENG C.A. MEY. AS MEMORY-ENHANCING AND ANTI-ANXIETY DRUGS" PROCEEDINGS OF THE BIENNIAL CONFERENCE OF THE CANADIAN SOCIETY FOR COMPUTATIONAL STUDIES OF INTELLIGENCE, XX, XX, vol. 47, no. 2, 1994, pages 121-124, XP009011374
- ESPINOLA E.B. ET AL: "Pharmacological activity of Guarana ( Paullinia cupana Mart.) in laboratory animals." JOURNAL OF ETHNOPHARMACOLOGY, (1997) 55/3 (223-229)., XP001159800

## Description

### BACKGROUND OF THE INVENTION

### 1. TECHNICAL FIELD

The invention relates to a dietary supplement in accordance with claim 1 consisting of
(i) an effective amount of an extract of the plant *Panax ginseng;* and
(ii) an effective amount of an extract of the plant *Paullinia cupana.*
(iii) one or more vitamins;
(iv) one or more minerals and/or trace elements; and
(v) optionnaly a carrier and/or adjuvant

### 2. BACKGROUND INFORMATION

The roots of *Panax ginseng* C.A. Meyer contain several triterpene glycosides named ginsenosides (or panaxosides) which are believed to contribute to the adaptogenic and physical performance enhancing properties of the ginseng extracts. It is used to treat anemia, diabetes mellitus, insomnia, neurasthenia, gastritis, abnormal blood pressure, dyspepsia, overstrain and fatigue.

The French patent application FR 2 712 191 claims and discloses compositions with anti-fatigue activity for facilitating weight loss, which contain an extract of ginseng root and an extract of *Guarana* seeds.

The French patent application FR 2 710 267 discloses an oral composition for treating male impotence consisting of ursodesoxycholic acid, *Panax ginseng* extract, *Muira puma* extract, yohimbine hydrochloride, *Guarana* extract, *Nux vomica* extract, *Damiana* extract, nicotinamide, nicotinic acid, tocopherol acetate, clenbuterol hydrochloride, and an excipient.

The UK patent application GB 2 285 578 discloses beverages containing guaranine in combination with other plant extracts including ginseng. The beverages enhance, *inter alia,* concentration and memory. It is a primary object of the present invention to provide a dietary supplement to improve the speed of memory and memory quality in normal, healthy subjects, to prevent deterioration of the speed of memory in people with decreased cognitive functions and to counteract cognitive fatigue.

### BRIEF SUMMARY OF THE INVENTION

Surprisingly, it has been found that the cognitive skills of a person can be improved by the administration of a dietary supplement consisting essentially of
(i) 50 to 100 mg of an extract of the plant *Paullinia cupana;*
(ii) a synergistically effective amount of an extract of the plant Panax ginseng having a weight ratio in the range of from 1:10 to 10:1 in relation to the extract of the plant Paullinia cupana;
(iii) one or more vitamins;
(iv) one or more minerals and/or trace elements; and
(v) optionally a carrier and/or adjuvant.

### DETAILED DESCRIPTION OF THE INVENTION

The two components (i) and (ii) together with the components (iii), (iv) and (v) are formulated together in one formulation. For example the formulation comprises the extract of dried *Panax ginseng* roots or other plant components, that optionally are powdered and the extract of dried *Paullinia cupana* seeds, or other plant components, that optionally are ground. The formulation may be in the form of tablets or The term "a person" or "a person in need thereof or "patient" as used hereinabove and hereinbelow relates to a healthy female or male person who is in need for an improvement of cognitive skills. As a rule such persons are adult or elderly people with an age of between 18 and 80, preferably between 20 and 65 years having an mean age of 42.5 years. The term "effective amount" as used herein means an amount sufficient to enhance the cognitive skills when component (i) and (ii) and (iii) and (iv) are administered together in one single dosage form. As a rule the combination of both the components (i) and (ii) shows a synergistic effect, which means that the effect on the cognitive skills is higher than expected from the mere additive effects of the single components (i) and (ii) and/or the single components (i), (ii), (iii) and (iv) alone.

Under the term "plant" is understood the plant itself as well as plant parts comprising the active ingredients. Like for example the leaves, the stems, the seeds, the fruits or roots as mentioned above. Preferably the plant or plant components are dried. Optionally, they may be cut to pieces, ground or powdered.

Under the term "extracts" are meant that the plants or plant components are extracted with a suitable solvent like water, ethanol, butanol, acetone, mixture thereof, ethers, oils or any other suitable solvent well known in the state of the art of extracting plants. These extracts can be used as such if pharmacologically acceptable or the solvent of the resulting solutions is removed and the residue is used as such or after further worked up, for example after resolving or re-suspending in a pharmacological suitable solvent.

Under the terms "principal active ingredients" and principal active substances are meant all active ingredients that are mainly responsible for the pharmacological effect. Preferably the formulation comprises all those ingredients of the plant of interest that are responsible for at least 75 per cent, more preferably at least 90 per cent of the pharmacological effect.

Under the term "enhancement of cognitive skills" are meant improvements of all kinds of situations, where a person is supposed to achieve an intellectual performance including quality, accuracy and speed of memory, learning capability, short and long term recollection, attention, alertness, as well as reduction of distress caused by high workload and/or the need to fulfill a demanding intellectual performance.

The term "dietary supplement" means a composition, which is for supplementing the regular food intake with additional nutritional elements to enhance quality of life, and which are freely available without prescription in groceries or super market, but not only in drugstores.

Preferably a *Ginseng* extract is used containing among other substances ginsenosides and polysaccharides, preferably containing at least 3 %, preferably 3.5 to 5.0 %, in particular 3.6 to 4.4% ginsenosides, most preferred is the standardized *Ginseng* extract under the Tradename G115®, which is commercially available from Pharmaton S.A., CH-6934 Bioggio, Switzerland.

*Guarana* extract is as a rule prepared from seeds of *Paullinia cupana.* This plant belongs to the *Sapindaceae,* a woody liana cultivated in the central Amazon's region. The most important components of *Paullinia cupana* are caffeine, tannins and saponins, and polysaccharides, disaccharides, fats and resins.

The seeds are carefully dried and ground to pieces of preferably 0.1 to 5.0 mm, in particular 0.5 to 1.5 mm. To achieve the desired content in caffeine the extraction is carried out with aqueous ethanol at elevated temperature, preferably at a temperature in the range of 60° to 80°C, over a time of at least 6 up to 10 hours. The preferred method is that of an exhaustive percolation with aqueous ethanol in the presence of a weak base. Preferably the ethanol is evaporated *in vacuo* and the semi-dry residue of the extraction is dried *in vacuo.* The resulting dry extract is preferably milled and sieved.

Preferably a dried extract of *Paullinia cupana* is used which contains up to 12 % caffeine.

In the present invention the weight ratio of *Ginseng* extract to *Guarana* extract ranges from 1 : 10 to 10 to 1, preferably from 1 : 5 to 2 :1, in particular from 1 : 3 to 1 : 1, and is most preferably about 1 : 1.9.

Preferably the invention relates to a method wherein said composition further comprises
(iii) one or more vitamins; and
(iv) one or more minerals and/or trace elements
in particular
wherein said one or more vitamins (iii) are selected from the group consisting of ProVitamin A, Vitamin B1, Vitamin B2, Vitamin B6, Vitamin B12, Vitamin C, Vitamin D3, d,1-alpha-Tocopherolacetate (Vitamin E), Folic Acid, Biotin (Vitamin H) and Vitamin PP; and/or
wherein said one or more minerals and/or trace elements (iv) are selected from the group consisting of Copper, Calcium, Iron, Zinc, Selenium, Phosphorus and Magnesium.

Preferably dietary supplement according to the invention consists essentially of
(i) 20 to 100 mg of an extract of the plant *Panax ginseng*;
(ii) 50 to 100 mg of an extract of the plant *Paullirzia cupana;*
(iii) 0.001 to 120 mg of one or more, preferably two to twelve, in particular five to eleven vitamins;
(iv) 0.01 to 400 mg of one or more, preferably two to ten, in particular four to seven minerals and/or trace elements; and
(v) optionally a carrier and/or adjuvant.

Most preferably the dietary supplement consists essentially of
30 to 50 mg, in particular about 40 mg of standardized *Ginseng* extract G115
60 to 90 mg, in particular about 75 mg of *Paullinia cupana* extract comprising up to 12 % by weight of caffeine
1000 to 1200 IU, in particular about 1100 IU of provitamin A (vitamin A activity)
1.0 to 2.0 mg, in particular about 1.4 mg of vitamin B1
1.0 to 2.0 mg, in particular about 1.6 mg of vitamin B2
1.0 to 3.0 mg, in particular about 2.0 mg of vitamin B6
0.5 to 2.0 µg, in particular about 1.0 µg of vitamin B12
10 to 100 mg, in particular about 60 mg of vitamin C
50 to 300 IU, in particular about 160 IU of vitamin D3
10 to 20 mg, in particular about 15 mg of d,1-alpha-tocopherolacetate (Vit. E)
100 to 300 µg, in particular about 200 µg of folic acid
100 to 200 µg, in particular about 150 µg of biotin
10 to 30 mg, in particular about 18 mg of vitamin PP
0.1 to 1.0 mg, in particular about 0.5 mg of copper
0 to 150 mg, in particular 0 or about 120 mg of calcium
1 to 10 mg, in particular about 5 mg of iron
1 to 10 mg, in particular about 5 mg of zinc
0 to 150 mg, in particular 0 or about 90 mg of phosphorus
10 to 150 µg, in particular about 30 µg of selenium
5 to 100 mg , in particular 10 to 70 mg of magnesium
and optionally a carrier and/or adjuvant.

Preferred is a dietary supplement which is formulated in the form of soft shell capsules or tablets.

Pre-selected amounts of the composition of the present invention containing the *Ginseng* extract (i), the Guarana extract (ii), vitamine(s) (iii) and minerals (IV) are preferably encapsulated in a soft gelatin including bovine, porcine, vegetable and succinylated gelatin shell. Optionally, the soft gelatin shell is essentially transparent so as to enhance the aesthetic qualities of the capsule. The soft gelatin shells as a rule comprise the following essential, as well as optional, components.
Gelatin is an essential component of the soft gelatin shells of the instant invention. The starting gelatin material used in the manufacture of soft capsules is obtained by the partial hydrolysis of collagenous material, such as the skin, white connective tissues, or bones of animals. Gelatin material can be classified as Type A gelatin, which is obtained from the acid-processing of porcine skins and exhibits an iso-electric point between pH 7 and pH 9; and Type B gelatin, which is obtained from the alkaline-processing of bone and animal (bovine) skins and exhibits an isoelectric point between pH 4.7 and pH 5.2. Blends of Type A and Type B gelatins can be used to obtain a gelatin with the requisite viscosity and bloom strength characteristics for capsule manufacture. Gelatin suitable for capsule manufacture is commercially available from the Sigma Chemical Company, St. Louis, Mo. For a general description of gelatin and gelatin-based capsules, see Remington's Pharmaceutical Sciences, 16th ed., Mack Publishing Company, Easton, Pa. (1980), page 1245 and pages 1576-1582; and U.S. Pat. No. 4,935,243, to Borkan et at., issued Jun. 19, 1990;

The soft gelatin shell of the capsules of the instant invention, as initially prepared, comprises from about 20% to about 60% gelatin, more preferably from about 25% to about 50% gelatin, and most preferably from about 40% to about 50% gelatin. The gelatin can be of Type & Type B, or a mixture thereof with bloom numbers ranging from about 60 to about 300.

A plasticizer is another component of the soft gelatin shells of the instant invention. One or more plasticizers is incorporated to produce a soft gelatin shell. The soft gelatin thus obtained has the required flexibility characteristics for use as an encapsulation agent. Useful plasticizers of the present invention include glycerin, sorbitan, sorbitol, or similar low molecular weight polyols, and mixtures thereof.

The shell of the present invention, as initially prepared, generally comprises from about 10% to about 35% plasticizer, preferably from about 10% to about 25% plasticizer, and most preferably from about 10% to about 20% plasticizer. A preferred plasticizer useful in the present invention is glycerin.

The soft gelatin shells of the instant invention also comprise water. Without being limited by theory, the water is believed to aid in the rapid dissolution or rupture of the soft gelatin shell upon contact with the gastrointestinal fluids encountered in the body.

The shell of the present invention, as initially prepared, generally comprises from about 15% to about 50% water, more preferably from about 25% to about 40% water, and most preferably from about 30% to about 40% water.

Other optional components which can be incorporated into the soft gelatin shells include colorings including color coatings, flavorings, preservatives, anti-oxidants, essences, and other aesthetically pleasing components.

The compositions of the present invention can be encapsulated within any conventional soft gelatin shell that is capable of substantially containing the composition for a reasonable period of time. The soft gelatin shells of the instant invention can be prepared by combining appropriate amounts of gelatin, water, plasticizer, and any optional components in a suitable vessel and agitating and/or stirring while heating to about 65 °C. until a uniform solution is obtained. This soft gelatin shell preparation can then be used for encapsulating the desired quantity of the fill composition employing standard encapsulation methodology to produce one-piece, hermetically-sealed, soft gelatin capsules. The gelatin capsules are formed into the desired shape and size so that they can be readily swallowed. The soft gelatin capsules of the instant invention are of a suitable size for easy swallowing and typically contain from about 100 mg to about 2000 mg of the active composition. Soft gelatin capsules and encapsulation methods are described in P. K. Wilkinson et at., "Softgels: Manufacturing Considerations", Drugs and the Pharmaceutical Sciences, 41 (Specialized Drug Delivery Systems), P. Tyle, Ed. (Marcel Dekker, Inc., New York, 1990) pp.409-449; F. S. Horn et at., "Capsules, Soft", Encyclopedia of Pharmaceutical Technology, vol. 2, J. Swarbrick and J. C. Boylan, eds. (Marcel Dekker, Inc., New York, 1990) pp. 269-284; M. S. Patel et at., "Advances in Softgel Formulation Technology", Manufacturing Chemist, vol. 60, no. 7, pp. 26-28 (July 1989); M. S. Patel et al., "Softgel Technology", Manufacturing Chemist, vol. 60, no. 8, pp. 47-49 (August 1989); R. F. Jimerson, "Softgel (Soft Gelatin Capsule) Update", Drug Development and Industrial Pharmacy (Interphex '86 Conference), vol. 12, no. 8 & 9, pp. 1133-1144 (1986); and W. R. Ebert, "Soft Elastic Gelatin Capsules: A Unique Dosage Form", Pharmaceutical Technology, vol. 1, no. 5, pp. 44-50 (1977); The resulting soft gelatin capsule is soluble in water and in gatrointestinal fluids. Upon swallowing the capsule, the gelatin shell rapidly dissolves or ruptures in the gastrointestinal tract thereby introducing the pharmaceutical actives from the liquid core into the physiological system.

Tablets of the invention will generally contain at least one pharmaceutically or dietary acceptable excipient conventionally used in the art of solid dosage form formulation.
Suitable excipients which may be incorporated include lubricants, for example magnesium stearate and stearic acid; disintegrants, for example cellulose derivatives; starches; binders, for example modified starches, polyvinylpyrrolidones and cellulose derivatives; glidants, for example colloidol silicas; compression aids, for example cellulose derivatives; as well as preservatives, suspending agents, wetting agents, flavoring agents, bulking agents, adhesives, coloring agents, sweetening agents appropriate to their form.

Suitably when the composition is in a tablet form, the composition will further comprise a film coat, e. g. hydroxypropylmethylcellulose (HPMC). Suitably the film coat is a transparent film coat, although an opaque film coat e. g. as obtained when using a film coat material in combination with an opacifier or a pigment such as titanium dioxide, a lake or a dye, may also be used. Advantageously it has been found that the inclusion of an opaque film coat minimizes tablet discoloration, which may occur on long-term storage of the tablet. Discoloration may also be avoided by incorporating a coloring agent into the tablet core. Suitably such tablets may also be film-coated, e. g. if desired for aesthetic purposes and/or to aid swallowing.

The extract is mixed with the excipients of the tablet core and compressed on a suitable tablet press.
The compression forces which are needed to produce tablets of suitable breaking resistance and hence with the required breakdown times are dependent on the shapes and sizes of the punching tools used. Compression forces in the range from 2 - 20 kN are preferred. Higher compression forces may lead to tablets with a delayed released of the active substances (i) to (iv). Lower compression forces may produce mechanically unstable tablets. The tablet cores may have different shapes; the preferred shapes are round biplanar or biconvex and oval or oblong forms.

The coating solution is prepared by mixing the film-forming agent with the colouring materials and a plasticizer in water. Using a suitable coating pan the film-coating solution is applied on to the tablet cores.

Preferably the tablets have an oblong shape to facilitate swallowing. In the case of a film-coated tablet containing 115 mg of the combined extracts (i) and (ii) an oblong tablet may be about 10-20 mm long and have a width of about 5 to 10 mm.

In another preferred embodiment of this invention the composition comprises vitamins, minerals, trace elements and caffeine in the form of an extract of guarana. The caffeine from the guarana extracts refreshes the body and the mental performance. It is milder than the caffeine usually obtained from coffee or tea.

Preferably, the dietary supplement according to the invention comprises the required dosis for daily intakeof each active ingredient in one package, in particular 0.05 to 1.00 gram of caffeine.

More preferably the dietary supplement according to the invention comprises at least five of the following components:
calcium phosphate, sodium dodecylsulfate, calcium hydrogenphosphate, magnesium oxide, potassium chloride, vitamin C, polyvinylpyrrolidone, nicotinicamide, iron(II) fumarate, vitamin E, zinc oxide, mangan sulfate, calcium pantothenate, stearinic acid, magnesium stearate, silicium dioxide, copper sulfate, vitamin B6, vitamin B2, lactose, vitamin B1, beta-carotine, vitamin A, chrome, sodium, folic acid, biotine, potassium iodide, sodium selenate, vitamin K1, vitamin D3, vitamin B12, vitamin B3 and vitamin B5;

By the addition Ginseng, an additional effect is achieved.

In order to allow the administration at the time of the real need, a product consisting of 2-6 tablets or capsules is prepared, which is integrated in a 7 x 12 cm package made of synthetic material. By this way of packaging the composition of vitamins caffeine and minerals can be carried eadily in the handluggage.

In order to verify the synergistic effect of the medication and /or dietary supplement of the present invention, a modified computerized version of the Serial Sevens test can be utilized. The original Serial Sevens test (M. Hayman, Arch. Neurol. Psychiatry 17: 125-130 (1947)) has appeared in a number of forms, including as part of the Mini-Mental State Examination [L.A. Taylor et al., J. Behav. Med. 11: 279-291 (1985)]. In the current study's novel computerized version participants are presented with a number from which they are instructed to serially subtract in sevens, entering their three digit responses on the keyboard number pad.

To first group of participants a placebo, to a second group a composition comprising *Ginseng* extract (i) and vitamins, to a third group a composition comprising *Guarana* extract (ii) and vitamins, and to a fourth group a composition comprising both *Ginseng* and *Guarana* extracts (i) and (ii) and vitamins have been administered.

The test duration is 2 minutes. A standard instruction screen informed the participant to count backwards in sevens from the given number, as quickly and accurately as possible, using the return key to enter each response. Participants are also instructed verbally that if they were to make a mistake they should carry on subtracting from the new incorrect number. Starting numbers between 800 and 999 are randomly generated, and are presented on the screen only until the first response has been made, after which time individual entered digits are represented by asterisks. The task is scored as total number of subtractions. The number of incorrect responses is also recorded. In the case of an error, subsequent responses are scored as positive if they are correct in relation to the new number.

The tests clearly show that the inventive composition increases the numbers of correct subtraction and reduces the mistakes made by the participants.

Furthermore, the efficacy of the inventive composition is tested in the following studies:
(A) To compare the quality of life (QoL) parameters in subjects receiving the inventive composition a double blind, placebo controlled, randomised, parallel group trial is carried out according to the method described in Drugs Exptl Clin Res 1996;22(6):323-329.
   The increase observed in the group receiving the inventive composition is statistically significantly superior to that seen in a conventional multivitamin group. Adverse effects of the compositions were minimal in both groups. Significant increases in body weight and in diastolic blood pressure were observed in the group treated with the conventional multivitamin preparation but not in the one treated with with the inventive composition, which is more effective than a conventional multivitamin preparation alone in improving quality of life in a population subjected to the stress of high physical and mental activity.
(B) To examine the influence of dietary supplements on health related quality of life (QoL) in a non-clinical population of 313 healthy adults. This study is the follow-up of the study published by Ussher et al., Brit J Health Psychol 5, 173-187 (2000).
   The inventive composition shows more significant improvement on physical strain, behavioural strain, cognitive strain and on overall strain.
(C) Another double-blind comparison of the effect on quality of life of a combination of vital substances including standardised Ginseng G115® and placebo is carried out according to the method described in Current Therapeutic Research 1994;55(1):32-42.
   Both the inventive composition and placebo improve the subjects' well-being, but the improvement from baseline is more pronounced in the group receiving the inventive composition. According to the Visual Analogue Scales scores, subjects receiving the inventive composition improve more significantly in overall score, alertness, relaxation, and appetite.

To enhance the cognitive skills, the capsule or tablet should be taken in dosages corresponding to 50 and 200 mg of the combined extract (i) and (ii), preferably 70-160 mg, in particular 90-130 mg daily. The total amount of extract may be divided up in 1 to 3 capsules or tablets a day, it is most preferably administered within one single dosage form. The daily dose should be taken at once, preferably in the morning.

The first successful enhancement of cognitive skills can be found shortly after the composition according to the present invention has been administered. However, impressive, sustainable improvement of cognitive skills can be expected within 6 weeks of continuous use. The optimum effect is maintained or amplified on longer use.

Procedures by way of example for preparing the compositions according to the invention will be described in more detail hereinafter. The Examples which follow serve solely as a detailed illustration without restricting the subject matter of the

### Example 1

### Method of manufacture of the Guarana extract preparation

The vegetable drug preparation, standardized *Guarana* extract (coded PC102), is prepared by the method hereafter:

| | | |
|---|---|---|
| 1. | Source plant: | *Paullinia Cupana* H.B. et Kunth |
| 2. | Part used: | dried seed |
| 3. | Extraction solvent: | ethanol (95-96% v/v): demineralized water (50 : 50) (v/v) |
| 4. | Ratio of vegetable drug to standardized extract: | limits 3 - 7 : 1 |
| 5. | Composition of the vegetable drug preparation: | 80 - 90% native extract |
| | | 20-10% maltodextrine MD01 |
| 6. | Standard batch size: 360 kg | |

### Production steps:

1. The dried seeds of *Paullinia Cupana* H.B. et Kunth are coarsely ground on a grinding machine (10 mm) to obtain fragments of about 0.5 - 1.5 mm size. the ground *Guarana* seeds are transferred to a percolator and extracted with ethanol 50%, alkalinized with sodium carbonate. The extraction procedure is repeated, under the same conditions, to exhaustion of the drug. As a rule five extractions lasting 3 hours each are needed. The exhaustion is considered achieved when the dried residue of the last percolate is < 0.5%.
2. The obtained percolates are partially concentrated *in vacuo* at a temperature below 50 °C.
3. Hydrochloric acid is added until a pH value of 5.5 - 6.5 is obtained.
4. Upon standing for 8 hours at 2 - 5 °C, the mixture is centrifuged.
5. The mixture is concentrated under vacuum, at a temperature below 50°C, until a soft extract is obtained which gives about 70% of dry residue.
6. The soft extract is dried under vacuum, at a temperature below 70°C.
7. The dry extract, is ground the total alkaloids content is determined and Maltodextrine MD01 is added, in order to obtain the required titer.
8. The product is ground with a centrifuge mill, mixed and sieved.

### Example 2

### Soft capsules

Soft gelatine capsules are prepared containing the following active ingredients:

| | Active ingredients | FORMULA |
|---|---|---|
| | | |
| Herbals | Ginseng Standardized Extract | 40 mg |
| | Paullinia cupana Extract | 75 mg |
| | PC102 | |
| Vitamins | ProVitamin A | 1110 IU (Vit A activity) |
| | Vitamin B 1 | 1.4 mg |
| | Vitamin B2 | 1.6 mg |
| | Vitamin B6 | 2.0 mg |
| | Vitamin B12 | 1.0 *µ*g |
| | Vitamin C | 60 mg |
| | Vitamin D3 | 160 IU |
| | d,1-alpha-Tocopherolacetate | 14.9 mg |
| | (Vit. E) | |
| | Folic Acid | 200 *µ*g |
| | Biotin | 150 *µ*g |
| | Vitamin PP | 18 mg |
| Minerals and | Copper | 0,5 mg |
| Trace elements | | |
| | Iron | 5 mg |
| | Zinc | 5 mg |
| | Selenium | 30 *µ*g |
| | Magnesium | 10 mg |

The ingredients are mixed and encapsulated into gelatine, water and a plasticifier to form a soft gelatine capsule.

### Example 3

**Tablets**

| Active ingredients | | FORMULA |
|---|---|---|
| | | |
| Herbals | Ginseng Standardized Extract | 40 mg |
| | Paullinia cupana Extract | 75 mg |
| | PC102 | |
| Vitamins | ProVitamin A | 1110 IU (Vit A activity) |
| | Vitamin B 1 | 1.4 mg |
| | Vitamin B2 | 1.6 mg |
| | Vitamin B6 | 2.0 mg |
| | Vitamin B 12 | 1.0 *µ*g |
| | Vitamin C | 60 mg |
| | Vitamin D3 | 160 IU |
| | d,1-alpha-Tocopherolacetate | 14.9 mg |
| | (Vit. E) | |
| | Folic Acid | 200 *µ*g |
| | Biotin | 150 *µ*g |
| | Vitamin PP | 18 mg |
| Minerals and | Copper | 0.5 mg |
| Trace elements | | |
| | Calcium | 120 mg |
| | Iron | 5 mg |
| | Zinc | 5 mg |
| | Phosphorus | 93 mg |
| | Selenium | 30 *µ*g |
| | Magnesium | 62,5 mg |

The ingredients are mixed with an excipient and compressed to a tablet.

### Example 4 (not according to the invention) 5

| Active ingredients | | FORMULA [ % ] |
|---|---|---|
| Herbals | Ginseng Standardized Extract | 15 |
| | Paullinia cupana Extract | 35 |
| | PC102 | |
| | Micro Algae | 15 |
| Vitamins, Minerals and Trace Elements | | 35 |

### Example 5 (not according to the invention)

| | Active ingredients | FORMULA [ % ] |
|---|---|---|
| Herbals | Paullinia cupana Extract | 55 |
| | PC102 | |
| | Micro Algae | 15 |
| Vitamins, Minerals and Trace Elements | | 30 |

## Claims

1. A dietary supplement consisting of
(i) 50 to 100 mg of an extract of the plant Paullinia cupana;
(ii) a synergistically effective amount or an extract of the plant Panax ginseng having a weight ratio in the range of from 1:10 to 10:1 in relation to the extract of the plant Paullinia cupana;
(iii) one or more vitamins;
(iv) one or more minerals and/or trace elements; and
(v) optionally a carrier and/or adjuvant.

2. A dietary supplement according to claim 1 consisting of
(i) 50 to 100 mg of an extract of the plant Paullinia cupana;
(ii) 20 to 60 mg of an extract of the plant Panax ginseng;
(iii) 0.001 to 120 mg of one or more vitamins;
(iv) 0.01 to 400 mg of one or more minerals and/or trace elements; and
(v) optionally a carrier and/or adjuvant.

3. A dietary supplement according to claim 1 consisting of
60 to 90 mg of Paullinia cupana extract
30 to 50 mg of standardized Ginseng extract
1000 to1200 IU of provitamin A
1.0 to 2.0 mg of vitamin B1
1.0 to 2.0 mg of vitamin B2
1.0 to 3.0 mg ofvitamin B6
1.0 to 2.0 µg of vitamin B12
10 to 100 mg of vitamin C
50 to 300 IU of vitamin D3
10 to 20 mg of d,1-alpha-tocopherolacetate (Vit. E)
100 to 300 µg of folic acid
100 to 200 µg of biotin
10 to 30 mg of vitamin PP
0.1 to 1.0 mg of copper
0 to 150 mg of calcium
1 to 10 mg of iron
1 to 10 mg of zinc
0 to 150 mg of phosphorus
50 to 150 µg of selenium
5 to 100 mg of magnesium
and optionally a carrier and/or adjuvant.

4. A dietary supplement according to any one of the preceding claims which is formulated in the form of soft shell capsules or tablets.

## Patentansprüche

1. Nahrungsergänzung, bestehend aus
(i) 50 bis 100 mg eines Extrakts der Pflanze Paullinia cupana;
(ii) einer synergistisch wirksamen Menge eines Extrakt der Pflanze Panax ginseng mit einem Gewichtsverhältnis im Bereich von 1:10 bis 10:1 im Verhältnis zu dem Extrakt der Pflanze Paullinia cupana;
(iii) ein oder mehrere Vitamine;
(iv) ein oder mehrere Mineralstoffe und/oder Spurenelemente; und
(v) gegebenenfalls einem Träger und/oder Hilfsstoff.

2. Nahrungsergänzung nach Anspruch 1, bestehend aus
(i) 50 bis 100 mg eines Extrakts der Pflanze Paullinia cupana;
(ii) 20 bis 60 mg eines Extrakts der Pflanze Panax ginseng;
(iii) 0,001 bis 120 mg von einem oder mehreren Vitamins;
(iv) 0,01 bis 400 mg von einem oder mehreren Mineralstoffen und/oder Spurenelementen; und
(v) gegebenenfalls einem Träger und/oder Hilfsstoff.

3. Nahrungsergänzung nach Anspruch 1, bestehend aus
60 bis 90 mg of Paullinia cupana-Extrakt
30 bis 50 mg standardisierter Ginseng-Extrakt
1000 bis 1200 IU Provitamin A
1,0 bis 2,0 mg Vitamin B1
1,0 bis 2,0 mg Vitamin B2
1,0 bis 3,0 mg Vitamin B6
1,0 bis 2,0 µg Vitamin B12
10 bis 100 mg Vitamin C
50 bis 300 IU Vitamin D3
10 bis 20 mg d,1-alpha-Tocopherolacetat (Vit. E)
100 bis 300 µg Folsäure
100 bis 200 µg Biotin
10 bis 30 mg Vitamin PP
0,1 bis 1,0 mg Kupfer
0 bis 150 mg Calcium
1 bis 10 mg Eisen
1 bis 10 mg Zink
0 bis 150 mg Phosphor
50 bis 150 µg Selen
5 bis 100 mg Magnesium
und gegebenenfalls einem Träger und/oder Hilfsstoff.

4. Nahrungsergänzung nach irgendeinem der vorhergehenden Ansprüche, die in Form von weichen Kapseln oder Tabletten formuliert ist.

## Revendications

1. Complément alimentaire consistant en
(i) 50 à 100 mg d'un extrait de la plante Paullinia cupana ;
(ii) une quantité efficace d'un point de vue synergique d'un extrait de la plante Panax ginseng présentant un rapport pondéral situé dans la plage de 1/10 à 10/1 par rapport à l'extrait de la plante Paullinia cupana ;
(iii) une ou plusieurs vitamines ;
(iv) un ou plusieurs minéraux et/ou oligo-éléments ; et
(v) éventuellement un support et/ou un adjuvant.

2. Complément alimentaire selon la revendication 1 consistant en
(i) 50 à 100 mg d'un extrait de la plante Paullinia cupana ;
(ii) 20 à 60 mg d'un extrait de la plante Panax ginseng ;
(iii) 0,001 à 120 mg d'une ou de plusieurs vitamines ;
(iv) 0,01 à 400 mg d'un ou de plusieurs minéraux et/ou oligo-éléments ; et
(v) éventuellement un support et/ou un adjuvant.

3. Complément alimentaire selon la revendication 1 consistant en
60 à 90 mg d'extrait de Paullinia cupana
30 à 50 mg d'extrait normalisé de Ginseng
1000 à 1200 UI de provitamine A
1,0 à 2,0 mg de vitamine B1
1,0 à 2,0 mg de vitamine B2
1,0 à 3,0 mg de vitamine B6
1,0 à 2,0 µg de vitamine B12
10 à 100 mg de vitamine C
50 à 300 UI de vitamine D3
10 à 20 mg d'acétate de d,1-alpha-tocophérol (vitamine E)
100 à 300 µg d'acide folique
100 à 200 µg de biotine
10 à 30 mg de vitamine PP
0,1 à 1,0 mg de cuivre
0 à 150 mg de calcium
1 à 10 mg de fer
1 à 10 mg de zinc
0 à 150 mg de phosphore
50 à 150 µg de sélénium
5 à 100 mg de magnésium
et éventuellement un support et/ou un adjuvant.

4. Complément alimentaire selon l'une quelconque des revendications précédentes qui est formulé sous la forme de capsules molles ou de comprimés.
